# EUROPÄISCHE PATENTSCHRIFT

(11) **EP 2 118 192 B1**
(45) Veröffentlichungstag und Bekanntmachung des Hinweises auf die Patenterteilung: **26.05.2010**
(21) Anmeldenummer: 08707351.6
(22) Anmeldetag: 29.01.2008
(51) Int. Cl.: C08K 5/00

(54) **WÄSSRIGE OLIGO- UND POLYESTERZUBEREITUNGEN**
AQUEOUS OLIGO- AND POLYESTER FORMULATIONS
PRÉPARATIONS AQUEUSES D'OLIGOESTERS ET DE POLYESTERS

(30) Priorität: 03.02.2007 DE 102007005532
(43) Veröffentlichungstag der Anmeldung: 18.11.2009
(73) Patentinhaber: Clariant Finance (BVI) Limited, Road Town, Tortola (VG)
(72) Erfinder: LANG, Frank-Peter, 65795 Hattersheim (DE); MORSCHHAEUSER, Roman, 55122 Mainz (DE)
(74) Vertreter: Hütter, Klaus
(86) Internationale Anmeldenummer: PCT/EP2008/000647
(87) Internationale Veröffentlichungsnummer: WO 2008/095626

(56) Entgegenhaltungen:
- EP-A- 1 454 949
- WO-A-01/42378
- WO-A-02/36579
- WO-A-02/062885
- WO-A-2006/012581
- DE-A1- 2 610 763
- DE-C1- 10 153 780
- FR-A- 2 856 693

## Beschreibung

Die Erfindung betrifft wässrige Formulierungen von Oligo- und Polyestern und deren Verwendung in Wasch- und Reinigungsmitteln, in der Textilindustrie und in Kosmetika.

Wasserlösliche oder wasserdispergierbare Polyester sind seit langem bekannt. Sie werden in der textilen Ausrüstung (Finishing) zur Hydrophilisierung, zur Verbesserung des Feuchtigkeitstransports, zur Verbesserung der Auswaschbarkeit von hydrophoben Anschmutzungen (Fetten und Ölen) und zur Verbesserung der Antistatik von Polyestergeweben eingesetzt. Bekannt ist auch deren Verwendung als sogenannte Soil Release Polymere in Wasch- und Reinigungsmitteln für Textilien. Hier dienen sie zur Verbesserung der Schmutzablösung von Synthesefasern, insbesondere von Polyester- und Polyestermischgeweben. Ferner ist die Anwendung bestimmter wasserlöslicher Oligo- bzw. Polyester in kosmetischen Zubereitungen wie Hautcremes oder Duschgelen bekannt. Hier dienen sie z.B. zur Verbesserung des Hautgefühls (skin conditioner).

Bei diesen wasserlöslichen oder wasserdispergierbaren Polyestern handelt es sich um Polykondensate auf der Basis von Dicarbonsäuren und Edukten, welche über zwei oder mehr Hydroxygruppen verfügen. Als Dicarbonsäure wird üblicherweise Terephthalsäure verwendet. Neben dieser können weitere zweibasische Carbonsäuren, wie z.B. die Isophthalsäure enthalten sein. Ferner können auch Tricarbonsäuren (als Vernetzer) eingesetzt werden. Als Edukte mit mehreren Hydroxygruppen (Polyole) werden z.B. Ethylenglykol, Propylenglykol, Butylenglykol, deren Dimere, Trimere, Oligomere oder Polymere eingesetzt. Auch Komponenten, welche über drei oder mehrere Hydroxygruppen verfügen, wie z.B. Glycerin oder Pentaerythrit können enthalten sein. Monofunktionelle Edukte, wie z.B. Methylpolyethylenglykole werden als Endcaps zur Steuerung des Molekulargewichts der Polyester eingesetzt.

Für die Verwendung der Polyester als Soil Release Polymere in flüssigen Wasch- und Reinigungsmitteln werden diese in unverdünnter Form, also als pastenförmige oder wachsartige Produkte, oder in der Form wässriger Zubereitungen angeboten.

Die unverdünnten Produkte haben den Nachteil, dass Sie zunächst geschmolzen werden müssen, damit sie anschließend in flüssiger, gieß- oder pumpbarer Form einer flüssigen Wasch- oder Reinigungsmittelformulierung zudosiert werden können.
Hierfür müssen aber beheizbare Lagerbehältnisse und Rohrleitungen bereitgestellt werden, was einen nicht unerheblichen technischen Aufwand darstellt.

Die handelsüblich verfügbaren wässrigen Zubereitungen haben wiederum den Nachteil, dass sie nur mit relativ niedrigen Wirkstoffgehalten herstellbar sind. Ein weiterer Nachteil aller wässrigen Polyesterzubereitungen ist, dass sie nur eine sehr beschränkte physikalische Lagerstabilität besitzen. Klare Zubereitungen, wie sie für die Herstellung klarer Wasch- und Reinigungsmittel unbedingt erforderlich sind, trüben bei erhöhten Temperaturen, wie sie im Sommer oder in südlichen Regionen vorherrschen, schon nach kurzer Zeit ein und es scheidet sich ein voluminöser, unansehnlicher Niederschlag ab. Sie können damit schon vor oder beim dem Transport zu dem Kunden unbrauchbar werden. Eine Abtrennung des Niederschlags mittels einer Filtration stellt dabei auch nur eine temporäre Problemlösung dar, da sich nachfolgend erneut ein solcher Niederschlag bilden kann. Die Bildung eines Niederschlags muss hier also unter allen Umständen vermieden werden. Wässrige Dispersionen neigen dagegen zur Phasentrennung, wobei die in Wasser dispergierten Polymerteilchen langsam sedimentieren.

In der PCT-Anmeldung 2005/006 344 wird die Verwendung von Polycarbonsäuren zur Stabilisierung von Oligo- und Polyesterformulierungen beschrieben.

Aufgabe der vorliegenden Erfindung ist es nun, wässrige Zubereitungen von Oligo- und Polyestern bereitzustellen, welche eine noch bessere physikalische Stabilität besitzen, also bei noch längerer Lagerung und höheren Temperaturen nicht eintrüben und von niedriger Viskosität sind und die somit vom Anwender einfach zu lagern und zu verarbeiten sind.

Es wurde nun überraschenderweise gefunden, dass dieses Ziel durch die Zugabe einer Phosphonsäure oder deren Salze erreicht werden kann.

Gegenstand der Erfindung sind wässrige Formulierungen von Oligo- und Polyestern, die im Wesentlichen 25 bis 90 Gew.-% eines Soil Release Polyesters und 0,1 bis 40 Gew.-% einer Phosphonsäure oder deren Salze enthalten.

Die Oligo- bzw. Polyester für die Herstellung der wässrigen Formulierungen können wasserlöslich oder wasserdispergierbar sowie nichtionisch oder anionisch sein. Die Molmasse dieser Polyester ist vorzugsweise kleiner gleich 20000, vorzugsweise kleiner gleich 10000 und besonders bevorzugt kleiner gleich 5000.

Nichtionische Oligo- und Polyester werden z. B. in den folgenden Patentschriften beschrieben: US 3,712,873, US 3,959,230, US 4,116,885, EP 0 442 101, DE 44 03 866, EP 253 567, EP 357 280 und DE 195 22 431. Sie können auf Basis der folgenden Monomere aufgebaut sein:

Zweiwertige Alkohole (Glykole), insbesondere Ethylenglykol; 1,2-Propylenglykol; 1,3-Propylenglykol; 1,2-Butylenglykol; 2,3-Butylenglykol; 1,4-Butylenglykol; Pentandiol; Hexandiol, 3-Methoxy-1,2-propylenglykol.

Mehrwertige Alkohole, insbesondere Glycerin, Pentaerythrit, Oligoglycerine sowie deren alkoxylierte Folgeprodukte.

Additionsprodukte, von bevorzugt 1 bis 5 mol Ethylenoxid und/oder Propylenoxid an 1 mol der oben genannten mindestens zweiwertigen Alkohole, wie z.B. Ethylendiglykol, Propylendiglykol, Additionsprodukte von bevorzugt 1 bis 3 mol Ethylenoxid und/oder Propylenoxid an 1 mol Glycerin, Additionsprodukte von bevorzugt 1 bis 4 mol Ethylenoxid und/oder Propylenoxid an Pentaerythrit.

Polyalkylenglykole. Diese leiten sich bevorzugt von Ethylenoxid, Propylenoxid, n-Butylenoxid oder lsobutylenoxid ab. Dabei kann es sich um Homopolymere, Copolymere oder Terpolymere der genannten Alkylenoxide handeln. Bei den Copolymeren kann es sich um Blockcopolymere, um statistische Copolymere oder um alternierende Copolymere handeln. Bevorzugt verwendet man Polyethylenglykol, Polypropylenglykol oder deren Blockcopolymere. Diese Polyalkylenglykole haben vorzugsweise Molmassen von bis zu 4000 g/mol.

Alkylpolyalkylenglykole, insbesondere wasserlösliche Anlagerungsprodukte von bevorzugt 5 bis 80 mol Alkylenoxid(en) an 1 mol C₈- bis C₂₄-Alkohole, C₆- bis C₁₈-Alkylphenole oder C₈- bis C₂₄-Alkylamine. Bevorzugte Alkylenoxide sind Ethylenoxid, Propylenoxid, Butylenoxid sowie deren Mischungen.

Aromatische Dicarbonsäuren, insbesondere Terephthalsäure, Isophthalsäure. Vorzugsweise enthalten die Polyester bis zu 60 Massen-%, vorzugsweise bis zu 50 Massen-% Terephthalsäure.

C₁-C₄-Alkylpolyalkylenglykole, wobei die hier zugrunde liegenden Polyalkylenglykole vorzugsweise Molmassen von bis zu 4000 g/mol aufweisen. Vorzugsweise kommt hier Methylpolyethylenglykol in Frage, das bis zu 90, vorzugsweise bis zu 50 und besonders bevorzugt bis zu 20 Einheiten Ethylenoxid enthält.

Aliphatische Dicarbonsäuren. Geeignete aliphatische Dicarbonsäuren enthalten z.B. 2 bis 10 Kohlenstoffatome. Beispiele hierfür sind Oxalsäure, Malonsäure, Bernsteinsäure, Glutarsäure, Adipinsäure, Maleinsäure, Fumarsäure, Itaconsäure und Citraconsäure. Sie können einzeln oder in Mischung eingesetzt werden.

Monohydroxymonocarbonsäuren, insbesondere Glykolsäure, Milchsäure, ω-Hydroxy-stearinsäure und ω-Hydroxycapronsäure.

Monocarbonsäuren, wie z.B. Benzoesäure, können als monofunktionelle Edukte zur Steuerung des Molekulargewichts eingesetzt werden.

Ester und Anhydride. Die vorgenannten Carbonsäuren können auch in der Form ihrer Ester oder, falls zugänglich, ihrer Anhydride eingesetzt werden. Beispiele hierfür sind Dimethyl-terephthalat, Diethyl-terephthalat, Oxalsäurediethylester, Adipinsäure-dimethylester, Phthalsäureanhydrid, Maleinsäureanhydrid, Bernsteinsäureanhydrid.

Anionische Oligo- und Polyester werden z.B. beschrieben in US 4,427,557; US 4,721,580; US 5,691,298; US 5,700,386; US 5,843,878; WO 96/18715; WO 95/02028; WO 95/02029 und EP 707627.

Zur Herstellung anionischer Oligo- und Polyester werden, neben den zuvor genannten, für die Herstellung nichtionischer Polyester verwendeten Komponenten, zusätzlich z.B. Hydroxyethansulfonsäure, Hydroxypropansulfonsäure, deren Umsetzungsprodukte mit Alkylenoxiden, bevorzugt mit Ethylenoxid und/ oder Propylenoxid, Glycerinsulfo-ethylether, Glycerinsulfopropylether, Sulfoisophthalsäure und Sulfobenzoesäure einkondensiert.

Oligo- und Polyester, die erfindungsgemäß eingesetzt werden können, haben vorzugsweise folgende Struktur: 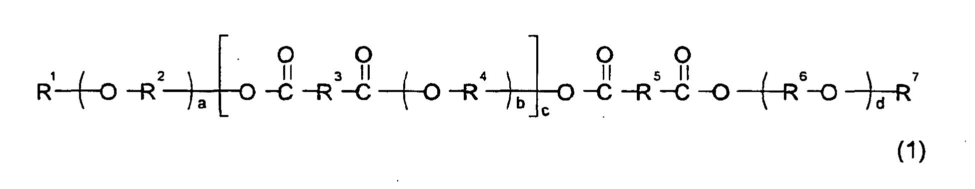 worin
- R¹ und R⁷: lineares oder verzweigtes C₁-C₁₈ Alkyl
- R², R⁴, R⁶: unabhängig voneinander Alkylen, bevorzugt Ethylen, Propylen, Butylen und/oder Cycloalkylen, z.B. 1,4-Cyclohexylen oder 1,4-Dimethylen-cyclohexylen sowie deren Gemische
- R³ und R⁵: Arylen oder Alkarylen, wie z.B. 1,4-Phenylen, 1,3-Phenylen, 1,2-Phenylen, 1,8-Naphthylen, 1,4-Naphthylen, 2,2'-Biphenylen, 4,4'-Biphenylen; Alkylen oder Alkenylen, wie z.B. Methylen, Ethylen, Propylen, Butylen, Pentylen, Hexylen; Cycloalkylen, wie z.B. Cyclohexylen
- a, b und d: eine Zahl zwischen 1 und 400
- c: eine Zahl zwischen 1 und 20.

Bevorzugt sind Oligo- und Polyester der Formel (1), worin
- R¹ und R⁷: Methyl und/ oder Ethyl,
- R², R⁴, R⁶: Ethylen, 1,2-Propylen, oder deren Gemische
- R³ und R⁵: 1,4-Phenylen und 1,3-Phenylen und
- a, b und d: eine Zahl zwischen 1 und 100
- c: eine Zahl zwischen 1 und 10
bedeuten.

Bevorzugte Oligo- bzw. Polyester sind auch solche der Formel (2) 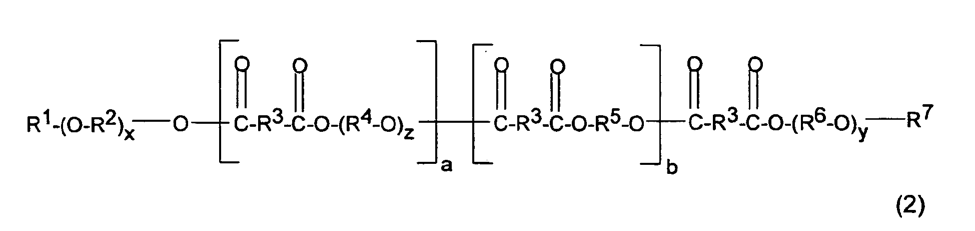 worin
R¹ und R⁷ lineares oder verzweigtes C₁-C₁₈-Alkyl, R² und R⁶ Ethylen, R³ 1,4-Phenylen, 1,3-Phenylen, R⁴ Ethylen, R⁵ Ethylen, 1,2-Propylen oder statistische Gemische von beliebiger Zusammensetzung von beiden, x und y unabhängig voneinander eine Zahl zwischen 1 und 500, z eine Zahl zwischen 10 und 140, a eine Zahl zwischen 1 und 12, b eine Zahl zwischen 7 und 40, bedeuten.

Bevorzugt bedeuten unabhängig voneinander in der Formel (2)

R¹ und R⁷ lineares oder verzweigtes C₁-C₄-Alkyl, x und y eine Zahl zwischen 3 und 45, z eine Zahl zwischen 18 und 70, a eine Zahl zwischen 2 und 5, b eine Zahl zwischen 8 und 12, a + b eine Zahl zwischen 12 und 35.

Die erfindungsgemäßen wässrigen Formulierungen enthalten neben den Oligo- und Polyestern zur Stabilisierung Phosphonsäuren oder deren Salze.

Als Phosphonsäuren bzw. deren Salze kommen vorzugsweise organische Phosphonsäuren oder deren Salze in Frage, vorzugsweise solche mit einer Molmasse von bis zu 20000 g/mol.

Als Phosphonsäuren kommen beispielsweise die folgenden Verbindungen in Frage: (Poly)phosphonsäure, Amino-trimethylenphosphonsäure (ATMP), Ethylendiamin-tetramethylenphosphon-säure, Hexamethylendiamin-tetra-methylenphosphonsäure, Diethylentriamin-pentamethylenphosphonsäure, Polyaminomethylenphosphonsäuren, Hexamethylentriaminpentamethylenphosphonsäure und Hydroxyethylidene-1,1-diphosphonsäure und (Poly)vinylphosphonsäure. Eine bevorzugte Phosphonsäure ist Hydroxyethylidene-1,1-diphosphonsäure (1).

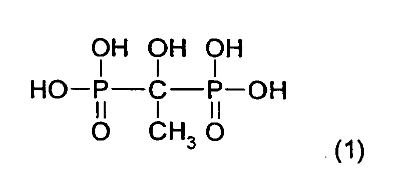

Bei den Salzen handelt es sich vorzugsweise um die Natrium-, Kalium-, Lithium-, Ammonium- oder substituierte Ammoniumsalze der genannten Phosphonsäuren.

Die erfindungsgemäßen Formulierungen können 25 bis 90 Gew.-%, bevorzugt 50 bis 85 Gew.%, besonders bevorzugt 60 bis 80 Gew.-% und ganz besonders bevorzugt 70 bis 80 Gew.-% der Oligo- bzw. Polyester enthalten. Der Gehalt an Phosphonsäure bzw. Phosphonat kann 0,1 bis 40 Gew.-%, bevorzugt 0,15 bis 25 Gew.-%, besonders bevorzugt 0,2 bis 10 Gew.-% und ganz besonders bevorzugt 0,25 bis 5 Gew.-% in der erfindungsgemäßen Formulierung betragen.

Die erfindungsgemäßen wässrigen Oligo- und Polyesterzubereitungen werden in der Regel in Konzentrationen von kleiner 5 Gew.-%, bevorzugt zu kleiner 3 Gew.-% und besonders bevorzugt zu 0,5 bis 1 Gew.-% in Wasch- und Reinigungsmittelformulierungen eingesetzt (Konzentrationsangaben hier bezogen auf den Polyesteranteil in der Zubereitung).

Die Wasch- und Reinigungsmittelformulierungen, in denen die erfindungsgemäßen wässrigen Oligo- und Polyesterzubereitungen eingesetzt werden können, sind bevorzugt Flüssigwaschmittel, Waschgele und Waschpasten sowie Weichspüler bzw. flüssige Wäscheconditioner, mit denen Textilien im Nachspülgang behandelt werden.
In diesen flüssigen Formulierungen können die genannten Polyesterzubereitungen mittels mechanischen Homogenisiereinrichtungen wie Rührern einfach eingearbeitet werden. Die wässrigen Polyesterzubereitungen können außerdem in speziellen Wäschebehandlungsmitteln, wie z.B. Flecklösern, Fleckensprays oder in Waschverstärkern verwendet werden.
Flüssige Formulierungen enthaltend die wässrigen Oligo- und Polyesterzubereitungen können in Folien verpackt sein, die entweder eine Schutzfunktion bei der Lagerung besitzen oder auch als Dosierhilfe dienen. Die Folien können wasserlöslich sein.

Die Wasch- und Reinigungsmittel bzw. Wäschebehandlungsmittel, welche die erfindungsgemäßen wässrigen Oligo- oder Polyesterzubereitungen enthalten, können darüber hinaus weitere übliche Bestandteile enthalten. Diese werden nachfolgend beschirieben:

### Anionische Tenside

Als anionische Tenside kommen Sulfate, Sulfonate, Carboxylate, Phosphate und Mischungen daraus in Betracht. Geeignete Kationen sind hierbei Alkalimetalle, wie z.B. Natrium oder Kalium oder Erdalkalimetalle, wie z.B. Calcium oder Magnesium sowie Ammonium, substituierte Ammoniumverbindungen, einschließlich Mono-, Di- oder Triethanolammoniumkationen, und Mischungen daraus.

Folgende Typen von anionischen Tensiden sind besonders bevorzugt: Alkylestersulfonate, Alkylsulfate, Alkylethersulfate, Alkylbenzolsulfonate, Alkansulfonate und Seifen, wie im Folgenden beschrieben.

Alkylestersulfonate sind unter anderem lineare Ester von C₈-C₂₀-Carboxylsäuren (d.h. Fettsäuren), welche mittels gasförmigem SO₃ sulfoniert werden, wie in "The Journal of the American Oil Chemists Society" 52 (1975), pp. 323-329 beschrieben wird.

Geeignete Ausgangsmaterialien sind natürliche Fette, wie z.B. Talg, Kokosöl und Palmöl, können aber auch synthetischer Natur sein.

Bevorzugte Alkylestersulfonate, speziell für Waschmittelanwendungen, sind Verbindungen der Formel 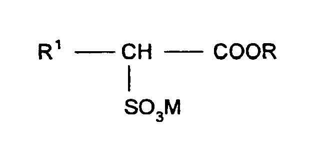 worin R¹ einen C₈-C₂₀-Kohlenwasserstoffrest, bevorzugt Alkyl, und R einen C₁-C₆ Kohlenwasserstoffrest, bevorzugt Alkyl, darstellt. M steht für ein Kation, das ein wasserlösliches Salz mit dem Alkylestersulfonat bildet. Geeignete Kationen sind Natrium, Kalium, Lithium oder Ammoniumkationen, wie Monoethanolamin, Diethanolamin und Triethanolamin. Bevorzugt bedeuten R¹ C₁₀-C₁₆-Alkyl und R Methyl, Ethyl oder Isopropyl. Besonders bevorzugt sind Methylestersulfonate, in denen R¹ C₁₀-C₁₆-Alkyl bedeutet.

Alkylsulfate sind hier wasserlösliche Salze oder Säuren der Formel ROSO₃M, worin R ein C₁₀-C₂₄-Kohlenwasserstoffrest, bevorzugt ein Alkyl- oder Hydroxyalkylrest mit C₁₀-C₂₀-Alkylkomponente, besonders bevorzugt ein C₁₂-C₁₈ Alkyl- oder Hydroxyalkylrest ist.
M ist Wasserstoff oder ein Kation, z.B. ein Alkalimetallkation (z.B. Natrium, Kalium, Lithium) oder Ammonium oder substituiertes Ammonium, z.B. Methyl-, Dimethyl- und Trimethyl-ammoniumkationen und quaternäre Ammoniumkationen wie Tetramethylammonium- und Dimethylpiperidiniumkationen und quartäre Ammoniumkationen, abgeleitet von Alkylaminen, wie Ethylamin, Diethylamin, Triethylamin und Mischungen davon.
Alkylketten mit C₁₂-C₁₆ sind für niedrige Waschtemperaturen (z.B. unter ca. 50°C) und Alkylketten mit C₁₆-C₁₈ für höhere Waschtemperaturen (z.B. oberhalb ca. 50°C) bevorzugt.

Alkylethersulfate sind wasserlösliche Salze oder Säuren der Formel RO(A)ₘ SO₃M, worin R einen unsubstituierten C₁₀-C₂₄-Alkyl- oder Hydroxyalkylrest, bevorzugt einen C₁₂-C₂₀ Alkyl- oder Hydroxyalkylrest, besonders bevorzugt C₁₂-C₁₈-Alkyl- oder Hydroxyalkylrest darstellt.

A ist eine Ethoxy- oder Propoxyeinheit, m ist eine Zahl größer als 0, vorzugsweise zwischen ca. 0,5 und ca. 6, besonders bevorzugt zwischen ca. 0,5 und ca. 3 und M ist ein Wasserstoffatom oder ein Kation, wie z.B. Natrium, Kalium, Lithium, Calcium, Magnesium, Ammonium oder ein substituiertes Ammoniumkation. Spezifische Beispiele von substituierten Ammoniumkationen sind Methyl-, Dimethyl-, Trimethylammonium- und quarternäre Ammoniumkationen wie Tetramethylammonium und Dimethylpiperidiniumkationen sowie solche, die von Alkylaminen, wie Ethylamin, Diethylamin, Triethylamin oder Mischungen davon abgeleitet sind. Als Beispiele seien C₁₂- bis C₁₈-Fettalkoholethersulfate genannt wobei der Gehalt an EO 1, 2, 2.5, 3 oder 4 mol pro mol des Fettalkoholethersulfats beträgt, und in denen M Natrium oder Kalium ist.

In sekundären Alkansulfonaten kann die Alkylgruppe entweder gesättigt oder ungesättigt, verzweigt oder linear und gegebenenfalls mit einer Hydroxylgruppe substituiert sein.

Die Sulfogruppe kann an einer beliebigen Position der C-Kette sein, wobei die primären Methylgruppen am Kettenanfang und Kettenende keine Sulfonatgruppen besitzen.
Die bevorzugten sekundären Alkansulfonate enthalten lineare Alkylketten mit ca. 9 bis 25 Kohlenstoffatomen, bevorzugt ca. 10 bis ca. 20 Kohlenstoffatome und besonders bevorzugt ca. 13 bis 17 Kohlenstoffatome. Das Kation ist beispielsweise Natrium, Kalium, Ammonium, Mono-, Di- oder Triethanolammonium, Calcium oder Magnesium, und Mischungen davon. Natrium als Kation ist bevorzugt.

Weitere geeignete anionische Tenside sind Alkenyl- oder Alkylbenzolsulfonate. Die Alkenyl- oder Alkylgruppe kann verzweigt oder linear und gegebenenfalls mit einer Hydroxylgruppe substituiert sein. Die bevorzugten Alkylbenzolsulfonate enthalten lineare Alkylketten mit ca. 9 bis 25 Kohlenstoffatomen, bevorzugt von ca. 10 bis ca. 13 Kohlenstoffatome, das Kation ist Natrium, Kalium, Ammonium, Mono-, Di- oder Triethanolammonium, Calcium oder Magnesium und Mischungen davon.

Für milde Tensidsysteme ist Magnesium als Kation bevorzugt, für Standardwaschanwendungen dagegen Natrium. Gleiches gilt für Alkenylbenzolsulfonate.

Der Begriff anionische Tenside schließt auch Olefinsulfonate mit ein, die durch Sulfonierung von C₈-C₂₄-, vorzugsweise C₁₄-C₁₆-a-Olefinen mit Schwefeltrioxid und anschließende Neutralisation erhalten werden. Bedingt durch das Herstellverfahren, können diese Olefinsulfonate kleinere Mengen an Hydroxyalkansulfonaten und Alkandisulfonaten enthalten.

Weitere bevorzugte anionische Tenside sind Carboxylate, z.B. Fettsäureseifen und vergleichbare Tenside. Die Seifen können gesättigt oder ungesättigt sein und können verschiedene Substituenten, wie Hydroxylgruppen oder α-Sulfonatgruppen enthalten. Bevorzugt sind lineare gesättigte oder ungesättigte Kohlenwasserstoffreste als hydrophober Anteil mit ca. 6 bis ca. 30, bevorzugt ca. 10 bis ca. 18 Kohlenstoffatomen.

Als anionische Tenside kommen weiterhin Salze von Acylaminocarbonsäuren in Frage, die durch Umsetzung von Fettsäurechloriden mit Natriumsarkosinat im alkalischen Medium entstehenden Acylsarcosinate; Fettsäure-Eiweiß-Kondensationsprodukte, die durch Umsetzung von Fettsäurechloriden mit Oligopeptiden erhalten werden; Salze von Alkylsulfamidocarbonsäuren; Salze von Alkyl- und Alkylarylethercarbonsäuren; Alkyl- und Alkenylglycerinsulfate wie Oleylglycerin-sulfate, Alkylphenolethersulfate, Alkylphosphate, Alkyletherphosphate, lsethionate, wie Acylisethionate, N-Acyltauride, Alkylsuccinate, Sulfosuccinate, Monoester der Sulfosuccinate (besonders gesättigte und ungesättigte C₁₂-C₁₈-Monoester) und Diester der Sulfosuccinate (besonders gesättigte und ungesättigte C₁₂-C₁₈-Diester), Acylsarcosinate, Sulfate von Alkylpolysacchariden wie Sulfate von Alkylpoly-glycosiden, verzweigte primäre Alkylsulfate und Alkylpolyethoxycarboxylate wie die der Formel RO(CH₂CH₂)ₖCH₂COO⁻M⁺, worin R C₈ bis C₂₂-Alkyl, k eine Zahl von 0 bis 10 und M ein Kation ist.

Nichtionische Tenside.

Kondensationsprodukte von aliphatischen Alkoholen mit ca. 1 bis ca. 25 mol Ethylenoxid.

Die Alkylkette der aliphatischen Alkohole kann linear oder verzweigt, primär oder sekundär sein, und enthält im Allgemeinen ca. 8 bis ca. 22 Kohlenstoffatome. Besonders bevorzugt sind die Kondensationsprodukte von C₁₀- bis C₂₀-Alkoholen mit ca. 2 bis ca. 18 mol Ethylenoxid pro mol Alkohol. Die Alkylkette kann gesättigt oder auch ungesättigt sein. Die Alkoholethoxylate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Typs sind Tergitol^{®} 5-S-9 (Kondensationsprodukt eines linearen sekundären C₁₁-C₁₅-Alkohols mit 9 mol Ethylenoxid), Tergitol^{®} 24-L-NMW (Kondensationsprodukt eines linearen primären C₁₂-C₁₄-Alkohols mit 6 mol Ethylenoxid bei enger Molgewichtsverteilung). Ebenfalls unter diese Produktklasse fallen die Genapol^{®}-Marken der Clariant GmbH.

Kondensationsprodukte von Ethylenoxid mit einer hydrophoben Basis, gebildet durch Kondensation von Propylenoxid mit Propylenglykol.

Der hydrophobe Teil dieser Verbindungen weist bevorzugt ein Molekulargewicht zwischen ca. 1500 und ca. 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic^{®}-Marken der BASF und die ^{®}Genapol PF-Marken der Clariant GmbH.

Kondensationsprodukte von Ethylenoxid mit einem Reaktionsprodukt von Propylenoxid und Ethylendiamin.

Die hydrophobe Einheit dieser Verbindungen besteht aus dem Reaktionsprodukt von Ethylendiamin mit überschüssigem Propylenoxid und weist im Allgemeinen ein Molekulargewicht von ca. 2500 bis 3000 auf. An diese hydrophobe Einheit wird Ethylenoxid bis zu einem Gehalt von ca. 40 bis ca. 80 Gew.-% Polyoxyethylen und einem Molekulargewicht von ca. 5000 bis 11000 addiert. Kommerziell erhältliche Beispiele dieser Verbindungsklasse sind die ^{®}Tetronic-Marken der BASF und die ^{®}Genapol PN-Marken der Clariant GmbH.

### Semipolare nichtionische Tenside

Diese Kategorie von nichtionischen Verbindungen umfasst wasserlösliche Aminoxide, wasserlösliche Phosphinoxide und wasserlösliche Sulfoxide, jeweils mit einem Alkylrest von ca. 10 bis ca. 18 Kohlenstoffatomen. Semipolare nichtionische Tenside sind auch Aminoxide der Formel

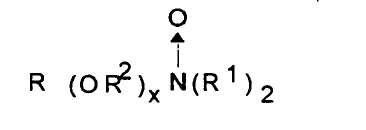

R ist hierbei eine Alkyl-, Hydroxyalkyl- oder Alkylphenolgruppe mit einer Kettenlänge von ca. 8 bis ca. 22 Kohlenstoffatomen, R² ist eine Alkylen- oder Hydroxyalkylengruppe mit ca. 2 bis 3 Kohlenstoffatomen oder Mischungen hiervon, jeder Rest R¹ ist eine Alkyl- oder Hydroxy-alkylgruppe mit ca. 1 bis ca. 3 Kohlenstoffatomen oder eine Polyethylenoxidgruppe mit ca. 1 bis ca. 3 Ethylenoxideinheiten und x bedeutet eine Zahl von 0 bis etwa 10. Die R¹-Gruppen können miteinander über ein Sauerstoff- oder Stickstoffatom verbunden sein und somit einen Ring bilden. Aminoxide dieser Art sind besonders C₁₀-C₁₈-Alkyldimethylaminoxide und C₈-C₁₂-Alkoxiethyl-Dihydroxyethylaminoxide.

### Fettsäureamide

Fettsäureamide besitzen die Formel 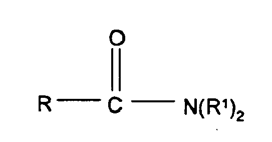 worin R eine Alkylgruppe mit ca. 7 bis ca. 21, bevorzugt ca. 9 bis ca. 17 Kohlenstoffatomen ist und jeder Rest R¹ Wasserstoff, C₁-C₄-Alkyl, C₁-C₄-Hydroxyalkyl oder (C₂H₄O)ₓH bedeutet, wobei x von ca. 1 bis ca. 3 variiert. Bevorzugt sind C₈-C₂₀-Amide, -monoethanolamide, -diethanolamide und -isopropanolamide.

Weitere geeignete nichtionische Tenside sind Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, alkoxylierte Triglycamide, Mischether oder Mischformyle, Alkyloligoglycoside, Alkenyloligoglycoside, Fettsäure-N-alkylglucamide, Phosphinoxide, Dialkylsulfoxide und Proteinhydrolysate.

Polyethylen-, Polypropylen- und Polybutylenoxidkondensate von Alkylphenolen.

Diese Verbindungen umfassen die Kondensationsprodukte von Alkylphenolen mit einer C₆- bis C₂₀-Alkylgruppe, die entweder linear oder verzweigt sein kann, mit Alkenoxiden. Bevorzugt sind Verbindungen mit ca. 5 bis 25 mol Alkenoxid pro mol Alkylphenol. Kommerziell erhältliche Tenside dieses Typs sind z.B. Igepal^{®} CO-630, Triton^{®} X-45, X-114, X-100 und X102, und die ^{®}Arkopal-N-Marken der Clariant GmbH. Diese Tenside werden als Alkylphenolalkoxilate, z.B. Alkylphenolethoxilate, bezeichnet.

### Zwitterionische Tenside

Typische Beispiele für amphotere bzw. zwitterionische Tenside sind Alkylbetaine, Alkylamidbetaine, Aminopropionate, Aminoglycinate, oder amphotere lmidazolinium-Verbindungen der Formel 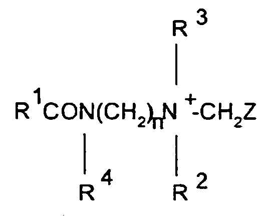 worin R¹ C₈-C₂₂-Alkyl- oder -Alkenyl, R² Wasserstoff oder CH₂CO₂M, R³ CH₂CH₂OH oder CH₂CH₂OCH₂CH₂CO₂M, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie Alkalimetall, Erdalkalimetall, Ammonium oder Alkanolammonium bedeutet.

Bevorzugte amphotere Tenside dieser Formel sind Monocarboxylate und Dicarboxylate. Beispiele hierfür sind Cocoamphocarboxypropionat, Cocoamidocarboxypropionsäure, Cocoamphocarboxyglycinat (oder auch als Cocoamphodiacetat bezeichnet) und Cocoamphoacetat.

Weitere bevorzugte amphotere Tenside sind Alkyldimethylbetaine (^{®}Genagen LAB/Clariant GmbH) und Alkyldipolyethoxybetaine mit einem Alkylrest mit ca. 8 bis 18 Kohlenstoffatomen und besonders bevorzugt mit ca. 12 bis ca. 18 Kohlenstoffatomen.

### Kationische Tenside

Geeignete kationische Tenside sind substituierte oder unsubstituierte geradkettige oder verzweigte quartäre Ammoniumsalze vom Typ R¹N(CH₃)₃⁺X⁻, R¹R²N(CH₃)₂⁺ X⁻, R¹R²R³N(CH₃) ⁺X⁻ oder R¹R²R³R⁴N⁺X⁻. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit ca. 1 bis ca. 4 C-Atomen, Phenyl, C₂- bis C₁₈-Alkenyl, C₇- bis C₂₄-Aralkyl, (C₂H₄O)ₓH, wobei x von ca. 1 bis ca. 3 bedeutet, ein oder mehrere Estergruppen enthaltende Alkylreste oder cyclische quartäre Ammoniumsalze sein. X ist ein geeignetes Anion.

### Builder (Gerüststoffe)

Builder können mit Gewichtsanteilen von etwa 5 % bis etwa 60 % in den Wasch- und Reinigungsmittelzusammensetzungen enthalten sein. Anorganische Builder umfassen beispielsweise Alkali-, Ammonium- und Alkanolammonium-salze von Polyphosphaten wie etwa Natriumtripolyphosphat, Natriumpyrophosphat und Natriumorthophosphat; Silikate; Carbonate einschließlich Bicarbonate und Sesquicarbonate; Sulfate und Aluminosilikate. Beispiele für Silikatgerüststoffe sind die Alkalimetallsilikate, insbesondere diejenigen mit einem SiO₂:Na₂O-Verhältnis zwischen 1,6:1 und 3,2:1. Aluminosilikatgerüststoffe sind ferner Zeolithe mit der Formel Na_{z}[(AlO₂)_{z}(SiO₂)y]-xH₂O, worin z und y ganze Zahlen von mindestens 6 bedeuten, das Verhältnis von z zu y zwischen 1,0 bis etwa 0,5 liegt und x eine ganze Zahl von etwa 15 bis etwa 264 bedeutet. Aluminosilikate können von kristalliner oder amorpher Struktur und natürlich vorkommend oder auch synthetisch hergestellt sein. Bevorzugte lonentauscher auf der Basis synthetischer kristalliner Aluminosilikate sind erhältlich unter der Bezeichnung Zeolith A, Zeolith P(B) und Zeolith X.

Wichtige organische Gerüststoffe (Cobuilder) sind Polycarboxylate, z.B. auf Basis von Acrylsäure und Maleinsäure (Sokalan CP-Marken/ BASF); Gerüststoffe auf Citratbasis, z.B. Citronensäure und ihre löslichen Salze, insbesondere Na-Citrat; Phosphonate, wie Ethan-1-hydroxy-1,1-diphosphonat und die anderen eingangs erwähnten Phosphonate.

Diese Phosphonate haben in Wasch- und Reinigungsmitteln die Funktion das Primärwaschvermögen gegenüber bestimmten Anschmutzungen zu verbessern. In bleichmittelhaltigen Waschmitteln verbessern sie die Lagerstabilität des Bleichmittels indem sie Schwermetallionen binden. Diese dem Stande der Technik entsprechende Aufgabe steht jedoch in keinem Zusammenhang mit dem erfindungsgemäßen Einsatz der Phosphonate in wässrigen Oligo- und Polyesterzubereitungen zur Verhinderung von Eintrübungen und Niederschlägen.

Weitere geeignete organische Gerüststoffe umfassen Polycarboxylverbindungen, wie beispielsweise Etherpolycarboxylate und Oxydisuccinate (wie beispielsweise in US 3,128,287 und US 3,635,830 beschrieben); "TMS/TDS"-Gerüststoffe (siehe US 4,663,071); Etherhydroxypolycarboxylate; Copolymere von Maleinsäureanhydrid mit Ethylen oder Vinylmethylether; Alkali-, Ammonium- und substituierte Ammoniumsalze von Polyessigsäuren, wie z.B. Ethylendiamintetraessigsäure und Nitrilotriessigsäure.

Die Wasch- und Reinigungsmittel und Wäschebehandlungsmittel, welche die wässrigen Oligo- bzw. Polyesterformulierungen enthalten können ferner die üblichen Hilfsstoffe enthalten, die die Reinigungswirkung verstärken, zur Pflege des zu waschenden Textils dienen oder die Gebrauchseigenschaften der Waschmittelzusammensetzung ändern. Geeignete Hilfsmittel umfassen beispielsweise Enzyme, insbesondere Proteasen, Lipasen, Cellulasen, Amylasen, Mannanasen, Glycosidasen; Enzymstabilisatoren; Schaumverstärker; Schaumbremsen; Anlauf- und/oder Korrosionsschutzmittel; Dispergatoren; Vergrauungsinhibitoren; Farbstoffe; Farbübertragungsinhibitoren; Füllmittel; optische Aufheller; Desinfektionsmittel; Alkalien; hydrotrope Verbindungen; Antioxidantien; Parfüme; Lösungsmittel; Lösungsvermittler; Verarbeitungshilfsmittel; Weichmacher und Antistatika.

Ferner können die Waschmittel, in denen die wässrigen Oligo- und Polyesterformulierungen eingesetzt werden, auch eines oder mehrere konventionelle Bleichmittel, Bleichaktivatoren, Bleichkatalysatoren und geeignete Stabilisatoren enthalten. Für Flüssigwaschmittel kommen zur Gewährleistung einer ausreichenden Lagerstabilität Zwei-Kammer-Flaschen zur Anwendung. Allgemein muss sichergestellt sein, dass die verwendeten Bleichmittel mit den Reinigungsmittelinhaltsstoffen verträglich sind. Prinzipiell können eingesetzt werden: Peroxysäuren, entweder als freie Peroxysäure, oder es kann eine Kombination aus a, einem anorganischen Persalz, beispielsweise Natriumperborat oder Natriumpercarbonat oder b, Wasserstoffperoxid mit einem organischen Peroxysäure-Vorläufer (Bleichaktivator) verwendet werden. Beispiele für Peroxysäuren umfassen die Peroxydodecandisäure (DPDA), das Nonylamid der Peroxybernsteinsäure (NAPSA), das Nonylamid der Peroxyadipinsäure (NAPAA) und Decyldiperoxybernsteinsäure (DDPSA), Nonanoyl-amidocaproyloxy-benzolsulfonsäure und Alkanoyloxybenzol-suffonsäuren wie die Nonanoyloxybenzolsulfonsäure (NOBS) und die Lauroyloxy-benzolsulfonsäure (LOBS) und Phthalimidoperoxycapronsäure (PAP). Besonders bevorzugt für die Anwendung der Oligo- und Polyesterformulierungen in Flüssigwaschmitteln und flüssigen Wäschebehandlungsmitteln in Zwei-Kammer-Flaschen sind Bleichsysteme auf Basis Wasserstoffperoxid und dem Bleichaktivator Tetraacetylethylendiamin (TAED).

Ein weiteres Anwendungsgebiet der erfindungsgemäßen Oligo- und Polyesterformulierungen sind die Behandlung (z.B. das Finishing) von Synthesefasern, insbesondere Polyesterfasern, oder Geweben, welche Synthesefasern, insbesondere Polyesterfasern, enthalten, in der Textilindustrie. Weiterhin können die erfindungsgemäßen Oligo- und Polyesterformulierungen auch in kosmetischen Zubereitungen wie Hautreinigungsmitteln, beispielsweise in Duschgelen, Schampoos, Seifen und in Hautpflegemitteln eingesetzt werden.

### Beispiele

1. Es wurde eine 70%-ige wässrige Zubereitung des Polyesters TexCare SRN-100 mit Zusatz von 0,5 Gew.-% (WS) der Phosphonsäure Dequest 2010 hergestellt. Dazu wurde der Polyester geschmolzen und in die Schmelze wurde das Wasser mit der vorgelösten Phosphonsäure eingerührt. Die Mischung wurde 15 Minuten nachgerührt und sodann unter Rühren auf Raumtemperatur abgekühlt.
Zur Untersuchung der thermischen Lagerstabilität wurde die Polyesterzubereitung bei 40°C eingelagert und über einen Zeitraum von 6 Monaten visuell beurteilt. Vergleichend wurde die Polyesterzubereitung ohne Zusatz der Phosphonsäure bzw. mit Zusatz von Sokalan CP 12 S hergestellt und beurteilt.

**Tabelle 1:**

| Stabilität einer wässrigen, 70 %igen Polyesterzubereitung mit Zusatz von 0,5 Gew.-% (Wirkstoff) Dequest 2010. Vergleichsbeispiel ohne Dequest 2010 bzw. mit Sokalan CP 12 S. | | | |
|---|---|---|---|
| 70 %ige Polyesterzubereitung aus TexCare SRN-100 | visuelle Bewertung | | |
| | nach der Herstellung | nach 3 Monaten | nach 6 Monaten |
| ohne Zusatz | klar, homogen | Bodensatz | Bodensatz |
| mit Sokalan CP 12 S | klar, homogen | klar, homogen | Trübung |
| mit Dequest 2010 | klar, homogen | klar, homogen | klar, homogen |

2. Es wurde eine 80 %ige wässrige Zubereitung des Polyesters TexCare SRN-100 mit Zusatz von 0,5 Gew.-% (Wirkstoff) der Phosphonsäure Dequest 2010 hergestellt. Dazu wurde der Polyester geschmolzen und in die Schmelze wurde das Wasser mit der vorgelösten Phosphonsäure eingerührt. Die Mischung wurde 1 Stunde nachgerührt und sodann unter Rühren auf Raumtemperatur abgekühlt.

Zur Untersuchung der thermischen Lagerstabilität wurde die Polyesterzubereitung bei 40°C eingelagert und nach 6 Monaten visuell beurteilt. Vergleichend wurde die Polyesterzubereitung ohne Zusatz der Phosphonsäure bzw. mit Zusatz von Sokalan CP 12 S hergestellt und beurteilt.

**Tabelle 2:**

| Stabilität einer wässrigen, 80%igen Polyesterzubereitung mit Zusatz von 0,5 Gew.-% (Wirkstoff) Dequest 2010. Vergleichsbeispiel ohne Dequest 2010 bzw. mit Sokalan CP 12 S. | | | |
|---|---|---|---|
| 80%ige Polyesterzubereitung aus TexCare SRN-100 | visuelle Bewertung | | |
| | nach der Herstellung | nach 3 Monaten | nach 6 Monaten |
| ohne Zusatz | klar, homogen | Bodensatz | Bodensatz |
| mit Sokalan CP 12 S | klar, homogen | klar, homogen | Trübung |
| mit Dequest 2010 | klar, homogen | klar, homogen | klar, homogen |

Verzeichnis der verwendeten Handelsnamen:

| | |
|---|---|
| TexCare^{®} SRN-1 00 (Clariant) | Nichtionischer Soil Release Polyester, 100 %ig. |
| | |
| Sokalan^{®} CP 12 S (BASF) | Acrylsäure-Maleinsäure-Copolymer, MM = 3000 g/mol, 50 %ig. |
| | |
| Dequest^{®} 2010 (Dequest) | Hydroxyethylidene-1,1-diphosphonsäure, 60 %ig. |

## Patentansprüche

1. Hochkonzentrierte wässrige Formulierungen von Oligo- und Polyestern, enthaltend im Wesentlichen 25 bis 90 Gew.-% eines Oligo- und Polyesters und 0,1 bis 40 Gew.-% einer Phosphonsäure oder deren Salze.

2. Formulierung gemäß Anspruch 1, wobei der Oligo- und Polyester ein wasserlöslicher oder wasserdispergierbarer Polyester ist.

3. Formulierung gemäß Anspruch 1, wobei der Oligo- und Polyester ein wasserlöslicher oder wasserdispergierbarer Polyester mit einer Molmasse von kleiner 20.000, bevorzugt von kleiner 10.000 und ganz besonders bevorzugt von kleiner 5.000 ist.

4. Formulierung gemäß Anspruch 1, wobei der Oligo- und Polyester ein wasserlöslicher oder wasserdispergierbarer Polyester ist, der durch Alkylpolyalkylenglykole endverschlossen ist.

5. Formulierung gemäß Anspruch 1, wobei der Oligo- und Polyester ein wasserlöslicher oder wasserdispergierbarer Polyester ist, der durch Methylpolyethylenglykole endverschlossen ist, wobei die Anzahl der Ethylenglykoleinheiten </= 90, bevorzugt </= 50 und besonders bevorzugt </= 20 beträgt.

6. Formulierung gemäß Anspruch 1, wobei der Oligo- und Polyester ein wasserlösliche oder wasserdispergierbarer Polyester ist, der </= 60 Massen-% und besonders bevorzugt </= 50 Massen-% veresterte Terephthalsäureeinheiten besitzt.

7. Formulierung gemäß Anspruch 1, wobei der Oligo- und Polyester ein wasserlöslicher oder wasserdispergierbarer Polyester ist, der Ethylenglykol- oder Polyethylenglykoleinheiten enthält.

8. Formulierung gemäß Anspruch 1, wobei der Oligo- und Polyester ein wasserlöslicher oder wasserdispergierbarer Polyester ist, der Propylenglykol- oder Polypropylenglykoleinheiten enthält.

9. Formulierung gemäß Anspruch 1, wobei der Oligo- und Polyester ein wasserlöslicher oder wasserdispergierbarer Polyester ist, der sowohl Ethylenglykol oder Polyethylenglykol als auch Propylenglykol- oder Polypropylenglykoleinheiten enthält.

10. Formulierung gemäß Anspruch 1, wobei der Oligo- und Polyester ein wasserlöslicher oder wasserdispergierbarer Polyester ist, der mittels einkondensierten Sulfoisophthalsäure- und/oder Glycerinsulfoethylethereinheiten und/ oder Glycerinsulfopropylethereinheiten anionisch modifiziert ist.

11. Formulierung gemäß Anspruch 1, wobei der Oligo- und Polyester ein wasserlöslicher oder wasserdispergierbarer Polyester ist, der mittels Hydroxyethansulfonsäure, Hydroxypropansulfonsäure oder deren Umsetzungsprodukten mit Ethylenoxid bzw. Ethylenglykol oder dessen Oligomeren und/oder Propylenoxid bzw. Propylenglykol oder dessen Oligomeren endverschlossen ist.

12. Formulierung nach Anspruch 1, enthaltend (Poly)phosphonsäure, Amino-trimethylenphosphonsäure (ATMP), Ethylendiamin-tetra-methylenphosphonsäure, Hexamethylendiamin-tetra-methylenphosphonsäure, Diethylentriamin-penta-methylenphosphonsäure, Polyaminomethylenphosphonsäure, Hexamethylentriamin-penta-methylenphosphonsäure, (Poly)vinylphosphonsäure oder 1-Hydroxyethylidene-1,1-diphosphonsäure oder deren Li-, Na-, K-, Ammonium- oder substituierten Ammoniumsalze.

13. Formulierung nach Anspruch 1, enthaltend 25 bis 90 Gew.-%, bevorzugt 50 bis 85 Gew.-%, besonders bevorzugt 60 bis 80 Gew.-% und ganz besonders bevorzugt 70 bis 80 Gew.-% der Oligo- bzw. Polyester.

14. Formulierung nach Anspruch 1, enthaltend 0,1 bis 40 Gew.%, bevorzugt 0,15 bis 25 Gew.-%, besonders bevorzugt 0,2 bis 10 Gew.-% und ganz besonders bevorzugt 0,25 bis 5 Gew.-% Phosphonsäure oder deren Salze.

15. Wasch- und Reinigungsmittel, enthaltend eine Formulierung nach Anspruch 1.

16. Kosmetische Zubereitungen, enthaltend eine Formulierung nach Anspruch 1.

17. Textilhilfsmittel zur Behandlung von Synthesefasern, insbesondere Polyesterfasern oder von textilen Geweben enthaltend Synthesefasern, insbesondere Polyesterfasern, enthaltend eine Formulierung nach Anspruch 1.

## Claims

1. A highly concentrated aqueous formulation of oligo- and polyesters, comprising essentially 25 to 90% by weight of an oligo- and polyester and 0.1 to 40% by weight of a phosphonic acid or salts thereof.

2. The formulation as claimed in claim 1, where the oligo- and polyester is a water-soluble or water-dispersible polyester.

3. The formulation as claimed in claim 1, where the oligo- and polyester is a water-soluble or water-dispersible polyester with a molar mass of less than 20 000, preferably of less than 10 000 and very particularly preferably of less than 5000.

4. The formulation as claimed in claim 1, where the oligo- and polyester is a water-soluble or water-dispersible polyester which is terminally capped by alkyl polyalkylene glycols.

5. The formulation as claimed in claim 1, where the oligo- and polyester is a water-soluble or water-dispersible polyester which is terminally capped by methyl polyethylene glycols, where the number of ethylene glycol units is </= 90, preferably </= 50 and particularly preferably </= 20.

6. The formulation as claimed in claim 1, where the oligo- and polyester is a water-soluble or water-dispersible polyester which has </= 60% by mass and particularly preferably </= 50% by mass of esterified terephthalic acid units.

7. The formulation as claimed in claim 1, where the oligo- and polyester is a water-soluble or water-dispersible polyester which contains ethylene glycol or polyethylene glycol units.

8. The formulation as claimed in claim 1, where the oligo- and polyester is a water-soluble or water-dispersible polyester which contains propylene glycol or polypropylene glycol units.

9. The formulation as claimed in claim 1, where the oligo- and polyester is a water-soluble or water-dispersible polyester which contains both ethylene glycol or polyethylene glycol and also propylene glycol or polypropylene glycol units.

10. The formulation as claimed in claim 1, where the oligo- and polyester is a water-soluble or water-dispersible polyester which is anionically modified by means of condensed-in sulfoisophthalic acid and/or glycerol sulfoethyl ether units and/or glycerol sulfopropyl ether units.

11. The formulation as claimed in claim 1, where the oligo- and polyester is a water-soluble or water-dispersible polyester which is terminally capped by means of hydroxyethanesulfonic acid, hydroxypropanesulfonic acid or reaction products thereof with ethylene oxide or ethylene glycol or oligomers thereof and/or propylene oxide or propylene glycol or oligomers thereof.

12. The formulation as claimed in claim 1, comprising (poly)phosphonic acid, aminotrimethylenephosphonic acid (ATMP), ethylenediaminetetra-methylenephosphonic acid, hexamethylenediaminetetramethylenephosphonic acid, diethylenetriaminepentamethylenephosphonic acid, polyaminomethylenephosphonic acid, hexamethylenetriaminepentamethylenephosphonic acid, (poly)vinylphosphonic acid or 1-hydroxyethylidene-1,1-diphosphonic acid or Li, Na, K, ammonium or substituted ammonium salts thereof.

13. The formulation as claimed in claim 1, comprising 25 to 90% by weight, preferably 50 to 85% by weight, particularly preferably 60 to 80% by weight and very particularly preferably 70 to 80% by weight, of the oligo- and/or polyester.

14. The formulation as claimed in claim 1, comprising 0.1 to 40% by weight, preferably 0.15 to 25% by weight, particularly preferably 0.2 to 10% by weight and very particularly preferably 0.25 to 5% by weight, of phosphonic acid or salts thereof.

15. A detergent or cleaner comprising a formulation as claimed in claim 1.

16. A cosmetic preparation comprising a formulation as claimed in claim 1.

17. A textile auxiliary for the treatment of synthetic fibers, in particular polyester fibers and of textile fabrics containing synthetic fibers, in particular polyester fibers, comprising a formulation as claimed in claim 1.

## Revendications

1. Compositions aqueuses d'oligo- et polyesters très concentrées, contenant essentiellement de 25 à 90 % en poids d'un oligo- ou polyester et de 0,1 à 40 % en poids d'un acide phosphonique ou de ses sels.

2. Composition selon la revendication 1, dans laquelle l'oligo- ou polyester est un polyester hydrosoluble ou dispersable dans l'eau.

3. Composition selon la revendication 1, dans laquelle l'oligo- ou polyester est un polyester hydrosoluble ou dispersable dans l'eau, ayant une masse moléculaire inférieure à 20 000, de préférence inférieure à 10 000 et de façon tout particulièrement préférée, inférieure à 5 000.

4. Composition selon la revendication 1, dans laquelle l'oligo- ou polyester est un polyester hydrosoluble ou dispersable dans l'eau, qui est coiffé en bout de chaîne par des alkylpolyalkylèneglycols.

5. Composition selon la revendication 1, dans laquelle l'oligo- ou polyester est un polyester hydrosoluble ou dispersable dans l'eau, qui est coiffé en bout de chaîne par des méthylpolyéthylèneglycols, le nombre des motifs éthylèneglycol étant ≤ 90, de préférence ≤ 50 et de façon particulièrement préférée, ≤ 20.

6. Composition selon la revendication 1, dans laquelle l'oligo- ou polyester est un polyester hydrosoluble ou dispersable dans l'eau, qui comporte ≤ 60 % en masse et de façon particulièrement préférée ≤ 50 % en masse de motifs acide téréphtalique estérifié.

7. Composition selon la revendication 1, dans laquelle l'oligo- ou polyester est un polyester hydrosoluble ou dispersable dans l'eau, qui contient des motifs éthylèneglycol ou polyéthylèneglycol.

8. Composition selon la revendication 1, dans laquelle l'oligo- ou polyester est un polyester hydrosoluble ou dispersable dans l'eau, qui contient des motifs propylèneglycol ou polypropylèneglycol.

9. Composition selon la revendication 1, dans laquelle l'oligo- ou polyester est un polyester hydrosoluble ou dispersable dans l'eau, qui contient aussi bien des motifs éthylèneglycol ou polyéthylèneglycol que des motifs propylèneglycol ou polypropylèneglycol.

10. Composition selon la revendication 1, dans laquelle l'oligo- ou polyester est un polyester hydrosoluble ou dispersable dans l'eau, qui est modifié par voie anionique au moyen de motifs acide sulfo-isophtalique et/ou sulfoéthyléther de glycérol et/ou de motifs sulfopropyléther de glycérol condensés.

11. Composition selon la revendication 1, dans laquelle l'oligo- ou polyester est un polyester hydrosoluble ou dispersable dans l'eau, qui est coiffé en bout de chaîne par de l'acide hydroxyéthanesulfonique, de l'acide hydroxypropane-sulfonique ou leurs produits de réaction avec l'oxyde d'éthylène ou l'éthylèneglycol ou leurs oligomères et/ou l'oxyde de propylène et/ou le propylèneglycol ou leurs oligomères.

12. Composition selon la revendication 1 contenant de l'acide (poly)phosphonique de l'acide amino-triméthylènephosphonique (ATMP), de l'acide éthylènediamine-tétraméthylènephosphonique, de l'acide hexaméthylènediamine-tétraméthylènephosphonique, de l'acide diéthylènetriamine-pentaméthylènephosphonique, de l'acide polyaminométhylènephosphonique, de l'acide hexaméthylènetriamine-pentaméthylènephosphonique de l'acide (poly)vinylphosphonique ou de l'acide 1-hydroxyéthylidène-1,1-diphosphonique ou leurs sels de Li, Na, K, ammonium ou ammonium substitué.

13. Composition selon la revendication 1, contenant de 25 à 90 % en poids, de préférence de 50 à 85 % en poids, de façon particulièrement préférée, de 60 à 80 % en poids et de façon tout particulièrement préférée, de 70 à 80 % en poids des oligo- ou polyesters.

14. Composition selon la revendication 1, contenant de 0,1 à 40 % en poids, de préférence de 0,15 à 25 % en poids, de façon particulièrement préférée, de 0,2 à 10 % en poids et de façon tout particulièrement préférée, de 0,25 à 5 % en poids d'acide phosphonique ou de ses sels.

15. Produit de lavage et de nettoyage, contenant une composition selon la revendication 1.

16. Préparations cosmétiques, contenant une composition selon la revendication 1.

17. Produit auxiliaire pour textile, destiné au traitement de fibres synthétiques, en particulier de fibres de polyester ou de tissus textiles contenant des fibres synthétiques, en particulier des fibres de polyester, contenant une composition selon la revendication 1.
